# EUROPEAN PATENT SPECIFICATION

(11) **EP 3 377 148 B1**
(45) Date of publication and mention of the grant of the patent: **30.06.2021**
(21) Application number: 16727539.5
(22) Date of filing: 05.05.2016
(51) Int. Cl.: A61M 5/20

(54) **AUTOINJECTOR**
AUTOINJEKTOR
AUTO-INJECTEUR

(30) Priority: 20.11.2015 CZ 20150822
(43) Date of publication of application: 26.09.2018
(73) Proprietor: ChemProtect.SK s.r.o., 039 01 Turcianske Teplice (SK); Leifr, Frantisek, 173 51 Unhost (CZ); Jedlicka, Tomás, 160 00 Praha (CZ)
(72) Inventor: POVRAZNIK, Josef, 16200 Praha 6 (CZ)
(74) Representative: Kendereski, Dusan
(86) International application number: PCT/IB2016/000582
(87) International publication number: WO 2017/085538

(56) References cited:
- WO-A1-2008/065646
- WO-A1-2009/103251
- WO-A2-03/039632
- DE-U1- 20 006 986
- GB-A- 770 341
- US-A1- 2004 138 611
- US-A1- 2013 274 707

## Description

### Field of the Invention

The invention is an autoinjector designed to deliver doses of medicine intramuscularly, particularly for the administration of active substances, so-called antidotes, in cases of poisoning by nerve agents. More specifically, it is an autoinjector used for the simultaneous administration of two or more drugs with a trigger mechanism, at the bottom of which there is an insert with two or more separate chambers and a septum at the bottom that is penetrable by an injection needle.

### Description of Related Art

Autoinjectors are used as a part of medical equipment of individuals in conditions where treatment by trained personnel is not accessible. The construction of the autoinjector is based on its use and the required dosage of active substances.

Based on the patent EP2221076 B1, the pneumatic powered autoinjector is known, in which pressurised gas acts against the piston in two stages and develops a different power at every stage, which is given by the size of the piston area which the gas affects in the particular stage.

From the published patent application CZ20080102 A1, a three-chamber autoinjector for delivering medicine is known. The mixing process and injection is controlled by guide elements in the shank rod of the autoinjector.

Thanks to the patent CZ303518 B6, the autoinjector composed of a hollow cylinder divided into three chambers by three pistons is known. A hypodermic injection needle is attached to the guide cylinder under the lower piston. The push mechanism comprises of a rod, compression spring and safety fuse. The safety fuse has two working positions and the user must shift the fuse to the second position, during which protrusion of the injection needle occurs.

The patent application WO 2009/103251 A1 discloses an injector allowing to mix, dissolve and apply medicaments stored in mutually separated chambers, consisting of a body where its inside contains a pushing mechanism and a part with three separated chambers with an injection needle. The three chambers are mutually separated by three pistons, and each chamber contains a medicament and/or a solvent. The second chamber contains a bypass connection. In the third chamber, the injection needle is fixed inside a guide cylinder located below the third piston, and the guide cylinder is equipped with bypasses connecting the third chamber with the entry of the injection needle. The third chamber is equipped with a second, lower section having a diameter by 5 to 10 % larger than diameters of the other chambers and is closed by an end cap. The part with three separated chambers is formed by a replaceable cartridge. The injector has a standby position and two operating positions, resulting in the administration of medicaments contained in three chambers to be sequential. In the first operating position, the content of the first chamber is mixed with the content of the second chamber by means of the bypass connection in the second chamber. In the second operating position, the content of the third chamber is administered at a first depth of the injection needle, and subsequently the medicinal solution from of the second and the first chambers is transferred into the third chamber and administered at a second depth of the injection needle. Such a multi-depth injection mode is disadvantageous when all medicaments contained in the injector are required to be administered at the same depth of the injection site. The patent application does not teach how to provide an injector with a single-depth injection mode.

The patent application US 2013/274707 A1 discloses a three-chambered autoinjector as well as the methods of administering medicaments to a human using the three-chambered autoinjector. The three chambers are separated by separation means - the first and the second chamber are separated by a separation assembly comprising a bypass and the second and the third chamber are separated by a separation plunger which permits the mixing by means of a widened bypass area. In certain embodiments, the autoinjector includes a first medicament in liquid form, a second medicament preferably in solid form, and a liquid composition for diluting the second medicament, and utilizes a three-chambered design so as to administer the first medicament and a solution comprising the second medicament and the liquid composition at different injection depths into the body of a human. The mixing of the second medicament and the liquid composition occurs in the second chamber by opening of the separation assembly bypass and the transfer of this mixture in the third chamber is permitted when the separation plunger is received in the widened bypass area. Such a multi-depth injection mode is disadvantageous when all medicaments contained in the injector are required to be administered at the same depth of the injection site. In an alternative embodiment an autoinjector is provided that delivers medicaments at the same injection depth. However, this alternative embodiment discloses only a plurality of separation assemblies without any widened bypass area, permitting the mode of mixing through a plurality of separation assembly bypasses only. The patent application does not teach how to provide an injector with a single-depth injection mode using a widened bypass area in all mixing steps.

The patent application WO 03/039632 A2 discloses a medicament container, medicament dispensing kit and packaging process that minimizes exposure of the medication to oxygen to prevent degradation of the medication. This disclosure teaches a single-compartment injector with a needle arranged externally with respect to the compartment, which results in a longer injector arrangement. The single compartment cannot be used for a plurality of medicaments requiring separate storage.

The patent application US 2004/138611 A1 discloses an automatic wet/dry medicament injector having a compartment for a dry medicament component and a compartment for a wet medicament component. The two compartments are separated by a seal structure that has a plug that is moved from a sealing position into a mixing position when the device is activated. The seal structure includes a wiper that scrapes the interior walls in the dry component compartment to prevent the dry component from accumulating at the seal/glass interface. A tapered insert funnels the mixed medicament components to an attached needle assembly, but it can be removed when the device is filled. A filter is provided between the medicament compartments and the needle assembly. A chamber between the filter and the needle assembly allows for better flow through the filter. An actuation assembly drives the plug into the mixing position and forces the mixed medicament through the needle and into the user. The needle is arranged externally with respect to the compartments, which results in a longer injector arrangement.

The patent application GB 770341 A discloses a hypodermic syringe comprising a fluid reservoir having a thin collapsible side wall inerging into a thicker rigid end wall and closed at its opposite end by a flat end wall. The hypodermic needle is disposed entirely within the reservoir with its butt end against the rigid end wall and its outlet end directed towards the flat end wall and a passage leading from the reservoir into the needle at its butt end. The flat end wall is sufficiently thin to be penetrated by the needle when the side wall of the reservoir is collapsed by forcing the end walls towards one another. The fluid reservoir presents only one compartment that cannot be used for a plurality of medicaments requiring separate storage.

The utility model DE 20006986 U1 discloses an injection device for injecting a liquid, in particular a medicament, into a subcutaneous tissue. The injection device has a chamber having an opening, an ampoule piston for reducing the volume of the chamber, a movable pressure piece which is biased by a spring element against a holding element, and a trigger for moving the holding element from a first position holding the pressure piece into a second position releasing the movement of the pressure piece. Furthermore, a base body is included for holding the trigger and for guiding the pressure piece in the holding element located in the second position against an end facing away from the chamber of the ampoule piston. The strength of the spring element and the holding element and/or the base body is dimensioned for use for a single or small number of injections. The chamber presents only one compartment that cannot be used for a plurality of medicaments requiring separate storage.

The patent application WO 2008/065646 A1 discloses an insertion device for a fluid delivery device for delivering a therapeutic fluid to a body of a patient and/or for sensing of a bodily analyte. The insertion device includes a housing and accommodated therein at least one penetrating cartridge provided with a penetrating member and with a subcutaneously insertable element. The insertion device is provided with a displacement mechanism which upon actuation is capable of protracting the penetrating cartridge towards the device for delivery and/or sensing. The protracting results in inserting of the penetrating cartridge into subcutaneous compartment of the body via an opening provided at the device for delivery and/or sensing and the insertion device allows evacuation of the penetrating member from the subcutaneous compartment via the opening. This disclosure teaches a single-compartment injector with a needle arranged externally with respect to the compartment, which results in a longer injector arrangement. The single compartment cannot be used for a plurality of medicaments requiring separate storage.

### The Principle of the Invention

The invention is defined in the appended claims. Based on the invention, an autoinjector for intramuscular self-administration of medical substances can be made. Though it is possible to deliver a number of different substances that may be self-administered using the autoinjector, it is meant primarily for the administration of active substances, so-called antidotes, in the case of nerve agent poisoning.

Administering antidotes usually entails administration of cholinesterase reactivators, e.g. pralidoxime, obidoxime or methoxime, and anticholinergic agents, such as atropine. Subsequently or simultaneously, it is suitable to apply an anticonvulsant with the administration of antidotes, for example diazepam. The autoinjector based on the invention has at least two, preferably three chambers, in which each drug is stored and then applied in one step.

The objective of the submitted invention is to increase reliability and comfort during the use of autoinjectors, and to ensure separate and sterile storage of medicaments until just before application. In the invention, this aim is reached by improved construction of the trigger mechanism, tight sealing of the individual chambers and adjusting the inner construction of the insert that enables intensive mixing of individual medical substances during application in one step.

A body of the autoinjector contains a trigger mechanism with a rod, a compression spring and a safety fuse which holds the rod in place with the compression spring. A cylindrical insert is placed in the lower part of the body of the autoinjector, under the trigger mechanism towards the bottom part of the autoinjector, which is the contact area for the user during application itself. The cylindrical insert is closed by a sliding seal with a plug at the top and with a septum penetrable by an injection needle at the bottom, and crimped with a metal cap with a hole to eject the injection needle. Furthermore, the insert contains at least one more sliding seal, which divides the insert into at least two chambers. Sliding seals are made of flexible material, e.g. suitable plastic or a butyl rubber and reinforced with a special plug holder or an injection needle. The lower chamber, placed between the septum and the sliding seal, has a diameter larger than the chamber placed above it. The placement and sizing of changes of the diameters in the lower chamber of the two-chamber insert are based on the viscosity of the solution of the active substance in the upper chamber. The above mentioned sliding seal is equipped with at least two, preferably four annular plates, which are sized so that they seal the contents of the upper chamber of active substances tightly. Meanwhile, in the lower chamber, the flow of solution of active substances is enabled through an annulus hole formed by the different diameters of the lower chamber and seal with simultaneous mixing. The seal with the needle holder and the injection needle is placed in the sliding seal, separating the lower chamber and the chamber above it. The upper part of the needle holder has a space connected with the lower chamber by one or more canals, through which the solution of active substances is transported from the lower chamber to the injection needle and then administered to the body. The lower part of the insert is sealed with a septum and crimped with a metal cap.

Inserts with more than two separate chambers contain two or more sliding seals, which are equipped with at least two, preferably four identical annular plates, that are sized so that they immaculately seal the contents of the three or more separate chambers. Mixing the solutions of the active substances occurs in the lower chamber thanks to the annulus, which is formed by the difference in diameters of the upper and lower chambers and the seal of the needle holder. The transport of the solution from the first chamber placed above the bottom mixing chamber, and then from the second and other chambers, gradually occurs through the annulus. The placement and sizing of the various diameters are based on the number of the chambers and viscosity of the solution of the active substances. The annular plates assure turbulent flow of the active substances, whereby their mixing is improved. In addition, the lower chamber serves as a mixing chamber of the active substances, wherein the resulting solution of active substances is transportable from the lower chamber to the injection needle and then administered in one step. The injection needle is attached to the needle holder (20) and secured against moving into the area above the injection needle by a valve. Such a single-depth mode of administration is especially advantageous for the particular combination of a cholinesterase reactivator, an anticholinergic agent and an anticonvulsant used in antidotes, since these active agents act synergistically and are therefore preferentially to be delivered at the same injection depth to maximize their synergistic effect as antidotes.

When using the autoinjector, the rod is moved along the axis of the body of the autoinjector and is simultaneously moved into the insert of the autoinjector. Thereby, a gradual slide of one of the seals with a plug in two-chamber inserts, or seals with an injection needle and a plug, occurs through the needle holder and the injection needle in the first stage of the shift. Air is expelled from the lower mixing chamber and mixes with the active substances and the solution is then pushed through the needle holder and the injection needle into the body. At the same time, the lower chamber serves as a mixing chamber, from which the solution of active substances is pushed through canals in the needle holder and further through the chamber above the injection needle into the injection needle. The mixing effect in the lower, mixing chamber is increased thanks to a turbulent flow of the solution through an annulus hole through the annular plates of the seals into the lower chamber along the walls of the lower chamber, which is enabled by the different diameters of the lower chamber in comparison to the chamber placed above.

A larger diameter of the lower chamber is a diameter of 1% - 9%, with an advantage of 4% to 8%, e.g. 5.6% larger than the diameter of the second, respectively third chamber. The flexibility of the material of the annular plates enables flow of the solutions of active substances. Thanks to increased mixing efficiency, the active substance may be placed in the bottom chamber, and, in the case of a nerve agent re-activator antidote, not only as a solution, but also as a crystalline form of the active substance.

The crimped metal cap allows to, even in the case of the present autoinjector, meet the standards for aseptic filling of drug vials prescribed by law. Aseptic filling of small volume solutions or substances in crystalline form is subject to the approval of the State Office for Drug Control or other state authorities allowing this activity.

In the convenient version, a safety fuse is arranged so that it can slide perpendicularly to the rod and a cotter pin secures it against inadvertent displacement. After the cotter pin is removed, analogically to a hand grenade, the autoinjector is ready for application and may be applied easily using a thumb grip.

### Overview of the Figures in Drawings

The invention is further described in detail in examples as shown in the drawings attached. The drawings depict:
- FIG. 1a: autoinjector based on one embodiment of the invention, with a threechamber insert, in a resting position;
- FIG. 1b: autoinjector based on FIG. 1a after application;
- FIG. 2a: detail of the safety fuse;
- FIG. 2b: detail of the upper part of the rod adjusted for mutual effect with the safety fuse;
- FIG. 3a: detail of the needle holder of the injection needle;
- FIG. 3b: detail of the seal between chambers;
- FIG. 4: detail of the lower part of the insert;
- FIG. 5: detail of the lower chamber and the chamber above it;
- FIG. 6a: a three-chamber insert of the autoinjector with a solution of active substances as in FIG. 1a;
- FIG. 6b: a two-chamber insert with a solution of active substances for the autoinjector based on other embodiments of the invention;
- FIG. 6c: a three-chamber insert of the autoinjector with active substances in the upper chamber and salts in a crystalline form in the lower chamber;
- FIG. 6d: a two-chamber insert with solutions of active substances in the upper chambers and salts in a crystalline form in the lower chamber based on anotherembodiment of the invention.

### Exemplary Embodiments of the Invention

### Example 1 (three-chamber "Triplepen" autoinjector)

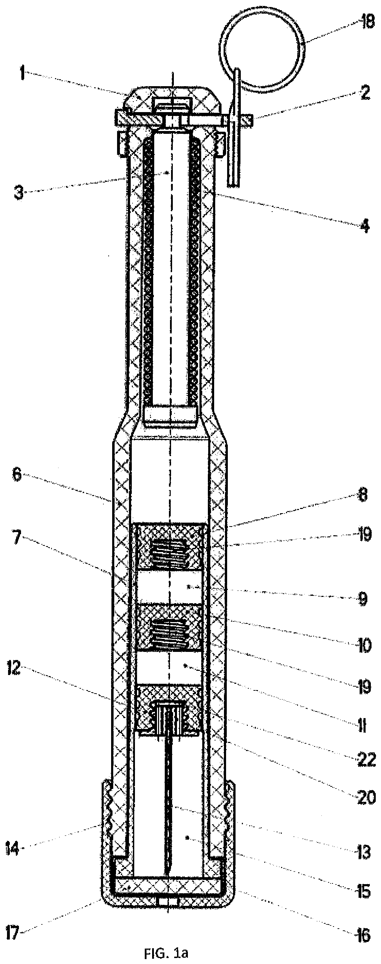

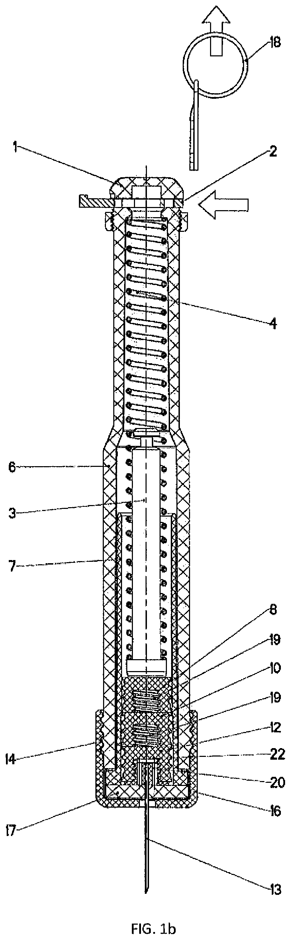

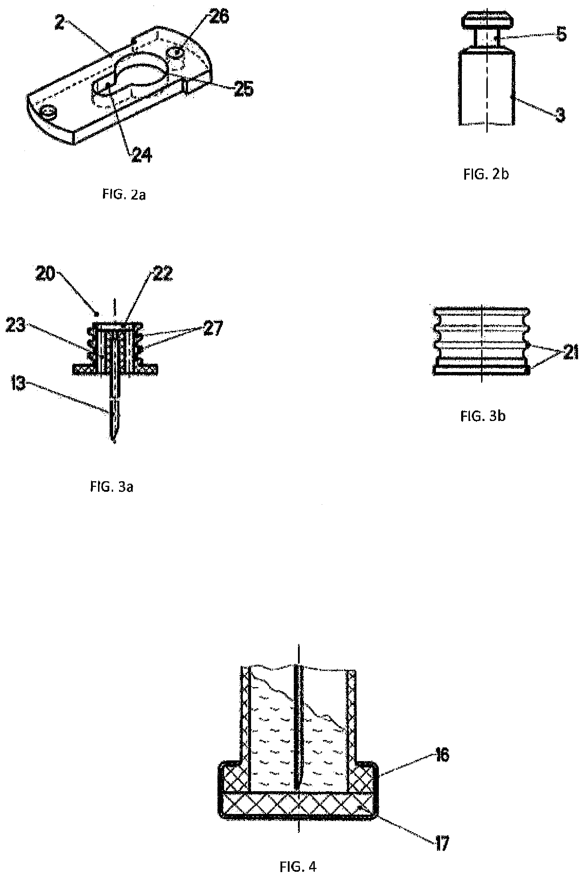

FIG. 1a shows the auto-injector with three mutually separate chambers 9, 11, 15. The upper part of the body 6 contains a trigger mechanism with a rod 3, a safety fuse 2 and a compression spring 4. In a resting position shown in FIG. 1a, the rod 3 secures the safety fuse 2 to hold the compression spring 4 in a compressed state. The safety fuse 2, shown in detail in Fig. 2a, holding the rod 3 against the force of the compressed spring 4 by a shank rod 5, which is captured in a narrow part 24 of the opening for the shank rod 5. By inserting the safety fuse 2 perpendicularly to the rod 3 to the left into the position of Fig. 1b, the shank rod 5 reaches the wide part 25 of the opening for the shank rod 5, through which the shank rod 5 passes through the rod and the spring 4 moves the trigger mechanism to the position shown in FIG. 1b. The safety fuse 2 is secured by a cotter pin 18, tucked in a hole 26 for the cotter pin.

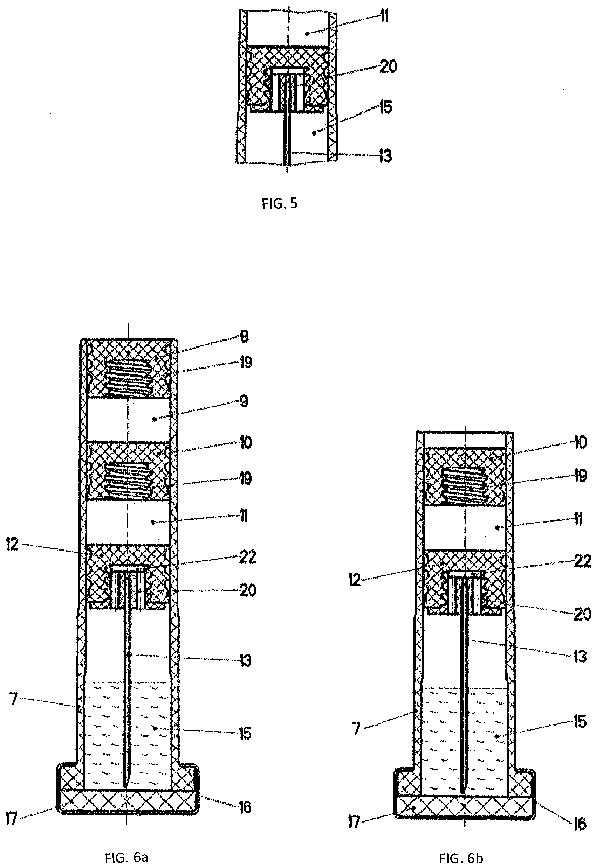

Below the trigger mechanism (data on its position is relative to the orientation of the images with the trigger mechanism at the top and the application end of the autoinjector at the bottom), a replaceable, hollow, cylindrical insert 7 is placed. The insert 7 is closed in its upper part by a sliding seal 8 with a plug 19 and at the bottom with a septum 17, which is penetrable by an injection needle 13, and an aluminium cap 16. The body of the insert is closed by a ferrule nut 14, with an opening for the injection needle 13. The insert 7 is divided by two other sliding seals 10, 12 into three chambers 9, 11, 15. As best seen in FIG. 5, the lower chamber 15 has a larger diameter than the remaining two chambers 9, 11, located above it. The sliding seals 8, 10, 12 are provided with annular plates 21. In the sliding seal 12, which delimits the lower chamber 15 from above and separates it from the chamber 11, located above it, a needle holder 20, provided with needle holder plates 27, is placed. In the needle holder 20, there is the injection needle 13, which is connected through the space 22 above the needle and through the canals 23 to the lower chamber 15, thus providing transport of active substance solution from the lower chamber into the needle and the body. The injection needle 13 is retained in the needle holder 20 and a valve secures it against displacement into the space 22 above the injection needle. Meanwhile, the lower chamber 15 serves as a mixing chamber from which the mixture is extruded through the canals 23 extending through the needle holder 20 and then through the space 22 above the injection needle into the injection needle 13.

The bottom of the insert 7 is provided with the septum 17 and is closed with the crimped aluminium cap 16, as shown in FIG. 4. The septum 17 is made, for example, of silicone rubber. However, it may also be made of polybutylene, natural rubber or another rubber type, and can be further modified, for example coated with polytetrafluoroethylene.

The sliding seals 8, 10, 12, as described in the embodiment, are each provided with four annular plates 21. The annular plates 21 in a resting position of the autoinjector, i.e. during storage, but also when wearing, prevent premature mixing of contents of adjacent chambers 9, 11; 11, 15. Once activating the autoinjector, the sliding seal 12 gets in the lower chamber 15, which has a somewhat larger diameter than the middle chamber 11 and the upper chamber 9, turbulent flow of the solution occurs through the annular plates 21 to the lower chamber 15 along the walls of the lower chamber 15.

In the example shown in Fig. 6a, the lower chamber 15 contains a solution of a cholinesterase reactivator. The middle chamber 11 in the described example contains a solution of an anticholinergic agent. The upper chamber 9 in the example described contains a solution of an anticonvulsant.

### Example 2 (two-chamber "Doublepen" autoinjector)

Containing an anticholinergic agent and a cholinesterase reactivator.

The triggering mechanism of the two-chamber autoinjector is the same as is described above for the three-chamber autoinjector. The only difference is that the force of the spring 7 via the rod 3 acts on the sliding seal 10, which closes the middle chamber 11. The arrangement of the insert 7 and the two-chamber and three-chamber autoinjector is illustrated in Fig. 6a, 6b. The same components have the same reference numerals as in Example 1.

As shown in Fig. 6B, the lower chamber 15 contains solution of a cholinesterase reactivator (an oxime), and the middle chamber 11, located above it, contains a solution of an anticholinergic agent.

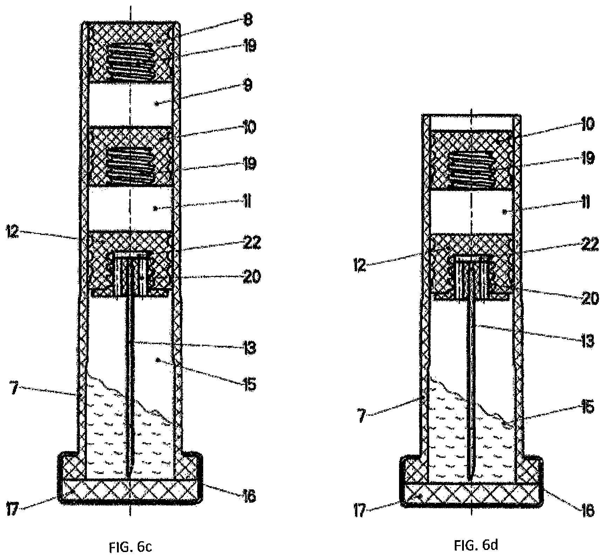

In other embodiments, there may be a solution of an oximein the lower chamber 15 (obidoxime, pralidoxime, methoxime, etc.), or alternatively, in a crystalline form, in particular Hl6 DMS, as shown in Fig. 6c and 6d.

### List of references

| | | | |
|---|---|---|---|
| 1 | cap | 15 | lower chamber |
| 2 | safety fuse | 16 | aluminium cap |
| 3 | rod | 17 | septum |
| 4 | spring | 18 | cotter pin |
| 5 | shank rod | 19 | plug |
| 6 | body of autoinjector | 20 | needle holder |
| 7 | insert | 21 | annular plates |
| 8 | sliding seal | 22 | area above the injection needle 13 |
| 9 | upper chamber | 23 | canal |
| 10 | sliding seal | 24 | narrow part of the opening for the shank rod 5 |
| 11 | middle chamber | | |
| 12 | sliding seal | 25 | wide part of the opening for the shank rod 5 |
| 13 | injection needle | | |
| 14 | ferrule nut | 26 | hole for cotter pin |
| | | 27 | needle holder plate |

## Claims

1. Autoinjector for simultaneous administration of two or more active substances in two or more separate chambers (9, 11, 15), having a body (6) with a trigger mechanism using a rod (3), a compression spring (4) and a safety fuse (2) which holds the rod (3) in place with the compression spring (4) in a compressed state, and a replaceable, cylindrical insert (7) that is located under the trigger mechanism, wherein the top of the insert (7) is closed with a sliding seal (8) having a plug (19) and the bottom of the insert (7) is closed with a septum (17) penetrable by an injection needle (13), wherein the insert (7) is further divided by at least one sliding seal (10, 12) into at least two chambers (9, 11, 15), wherein the lower chamber (15) has a larger diameter than the chamber (11) located above it, wherein the injection needle (13) held by a needle holder (20) is located in the sliding seal (12) separating the lower chamber (15) from the chamber (11) located above it, and wherein the injection needle (13) is liquidly connected via an area (22) above the injection needle (13) and via canals (23), whereby the sliding seals (8, 10, 12) are equipped with annular plates (21) providing for turbulent flow for improved mixing of the active substances, wherein the lower chamber (15) serves as a mixing chamber of the active substances, wherein the resulting solution of active substances is transportable from the lower chamber (15) through the canals (23) and further through the area (22) to the injection needle (13) and then administered in one step, and the insert (7) is crimped with a metal cap, and wherein the injection needle (13) is attached to the needle holder (20) and secured against moving into the area (22) above the injection needle (13) by a valve.

2. Autoinjector according to claim 1, **characterized in that** the safety fuse (2) is sliding and is placed in an upright position to the rod (3) and a cotter pin (18) prevents it from being pushed unintentionally, wherein the safety fuse (2) has a hole with a wide part (24) and a narrow part (25) to secure the rod (3) and its use.

3. Autoinjector according to claims 1 or 2, **characterized in that** the insert (7) is divided by the sliding seal (12) with the needle holder (20) into two chambers (11,15).

4. Autoinjector according to claim 3, **characterized in that** the upper chamber (11) contains an anticholinergic agent and the lower chamber contains a cholinesterase reactivator.

5. Autoinjector according to claims 1 and 2, **characterized in that** the insert (7) is divided by the sliding seal (10) and the sliding seal (12) with the needle holder (20) into three chambers (9, 11, 15).

6. Autoinjector according to claim 5, **characterized in that** the upper chamber (9) contains an anticonvulsant, the middle chamber (11) contains an anticholinergic agent and the lower chamber (15) contains a cholinesterase reactivator.

7. Autoinjector according to any of the above mentioned claims, **characterized in that** the lower chamber (15) contains a cholinesterase reactivator in a dissolved form.

8. Autoinjector according to claims 1 to 6, **characterized in that** the lower chamber (15) contains a cholinesterase reactivator in a crystalline form.

## Patentansprüche

1. Ein Autoinjektor zur gleichzeitigen Verabreichung von zwei oder mehr Wirkstoffen in zwei oder mehr getrennten Kammern (9, 11, 15), umfassend einen Körper (6) mit einem Auslösemechanismus, der eine Stange (3), eine Druckfeder (4) und eine Sicherung (2) umfasst, die die Stange (3) mit der Druckfeder (4) in einem zusammengedrückten Zustand hält, und einen auswechselbaren, zylindrischen Einsatz (7), der unter dem Auslösemechanismus angeordnet ist, wobei die Oberseite des Einsatzes (7) mit einer Gleitdichtung (8) mit einem Stecker (19) verschlossen ist, und die Unterseite des Einsatzes (7) mit einem Septum (17) der von einer Injektionsnadel (13) durchdringbar ist, verschlossen ist, wobei der Einsatz (7) durch mindestens eine Gleitdichtung (10, 12) in mindestens zwei Kammern (9, 11, 15) unterteilt ist, wobei die untere Kammer (15) einen größeren Durchmesser aufweist als die darüber liegende Kammer (11), wobei die von einem Nadelhalter (20) gehaltene Injektionsnadel (13) sich in der Gleitdichtung (12), die die untere Kammer (15) von der darüber liegenden Kammer (11) trennt, befindet, und wobei die Injektionsnadel (13) über einen Bereich (22) oberhalb der Injektionsnadel (13) und über Kanäle (23) flüssigkeitsleitend verbunden ist, wobei die Gleitdichtungen (8, 10, 12) mit Ringplatten (21) ausgestattet sind, um einer turbulenten Strömung zur verbesserten Vermischung der Wirkstoffe zu verfügen, wobei die untere Kammer (15) als Mischkammer der Wirkstoffe dient, wobei die resultierende Wirkstofflösung aus der unteren Kammer (15) durch die Kanäle (23) und weiter durch den Bereich (22) zur Injektionsnadel (13) transportierbar ist und dann in einem Schritt verabreicht wird, und der Einsatz (7) mit einer Metallkappe geriffelt ist, und wobei die Injektionsnadel (13) an dem Nadelhalter (20) befestigt und durch ein Ventil gegen eine Bewegung in den Bereich (22) oberhalb der Injektionsnadel (13) gesichert ist.

2. Der Autoinjektor nach Anspruch 1, **dadurch gekennzeichnet, dass** die Sicherung (2) gleitend ist und in aufrechter Lage mit der Stange (3) gelagert ist und ein Splint-Stift (18) ein unbeabsichtigtes Einschieben von der Sicherung (2) verhindert, wobei die Sicherung (2) ein Loch mit einem breiten Teil (24) und einem schmalen Teil (25) zur Sicherung der Stange (3) und deren Verwendung aufweist.

3. Der Autoinjektor nach Anspruch 1 oder 2, **dadurch gekennzeichnet, dass** der Einsatz (7) durch die Gleitdichtung (12) mit dem Nadelhalter (20) in zwei Kammern (11, 15) unterteilt ist.

4. Der Autoinjektor nach Anspruch 3, **dadurch gekennzeichnet, dass** die obere Kammer (11) ein Anticholinergikum enthält und die untere Kammer einen Cholinesterase-Reaktivierer enthält.

5. Der Autoinjektor nach Anspruch 1 und 2, **dadurch gekennzeichnet, dass** der Einsatz (7) durch die Gleitdichtung (10) und die Gleitdichtung (12) mit dem Nadelhalter (20) in drei Kammern (9, 11, 15) unterteilt ist.

6. Der Autoinjektor nach Anspruch 5, **dadurch gekennzeichnet, dass** die obere Kammer (9) ein Antikonvulsivum enthält, die mittlere Kammer (11) ein Anticholinergikum enthält und die untere Kammer (15) einen Cholinesterase-Reaktivierer enthält.

7. Der Autoinjektor nach einem der vorhergehenden Ansprüche, **dadurch gekennzeichnet, dass** die untere Kammer (15) einen Cholinesterase-Reaktivierer in gelöster Form enthält.

8. Der Autoinjektor nach den Ansprüchen 1 bis 6, **dadurch gekennzeichnet, dass** die untere Kammer (15) einen Cholinesterase-Reaktivierer in kristalliner Form enthält.

## Revendications

1. Un auto-injecteur pour l'administration simultanée d'au moins deux substances actives dans au moins deux chambres séparées (9, 11, 15), comprenant un corps (6) avec un mécanisme de déclenchement utilisant une tige (3), un ressort de compression (4) et un fusible de sécurité (2) qui maintient la tige (3) en place avec le ressort de compression (4) dans un état comprimé, et un insert cylindrique remplaçable (7) qui est situé sous le mécanisme de déclenchement, où la partie supérieure de l'insert (7) est fermée par un joint coulissant (8) ayant une fiche (19) et la partie inférieure de l'insert (7) est fermée par un septum (17) pénétrable par une aiguille d'injection (13), où l'insert (7) est en outre divisé par au moins un joint coulissant (10, 12) en au moins deux chambres (9, 11, 15), où la chambre inférieure (15) présente un diamètre supérieur à celui de la chambre (11) située au-dessus de celle-ci, où l'aiguille d'injection (13) maintenue par un porte-aiguille (20) est située dans le joint coulissant (12) qui sépare la chambre inférieure (15) à partir de la chambre (11) située au-dessus de celle-ci, et où l'aiguille d'injection (13) est reliée de manière fluidique par l'intermédiaire d'une zone (22) au-dessus de l'aiguille d'injection (13) et par l'intermédiaire de canaux (23), où les joints coulissants (8, 10, 12) sont équipés de plaques annulaires (21) fournissant un écoulement turbulent pour un mélange amélioré des substances actives, où la chambre inférieure (15) serve de chambre de mélange des substances actives, où la solution résultante de substances actives peut être transportée de la chambre inférieure (15) à travers les canaux (23) et à travers la zone (22) jusqu'à l'aiguille d'injection (13), et puis administrée en une étape, et l'insert (7) est serti avec un capuchon métallique, et où l'aiguille d'injection (13) est fixée au porte-aiguille (20) et fixée contre le déplacement dans la zone (22) au-dessus de l'aiguille d'injection (13) par une soupape.

2. L'auto-injecteur selon la revendication 1, **caractérisé en ce que** le fusible de sécurité (2) est coulissant et placé en position verticale sur la tige (3) et une goupille de clavette (18) l'empêche d'être poussé de manière non intentionnelle, où le fusible de sécurité (2) comprend un trou avec une partie large (24) et une partie étroite (25) pour fixer la tige (3) et son utilisation.

3. L'auto-injecteur selon les revendications 1 ou 2, **caractérisé en ce que** l'insert (7) est divisé par le joint coulissant (12) avec le porte-aiguille (20) en deux chambres (11, 15).

4. L'auto-injecteur selon la revendication 3, **caractérisé en ce que** la chambre supérieure (11) contient un agent anticholinergique et la chambre inférieure contient un réactivateur de cholinestérase.

5. L'auto-injecteur selon les revendications 1 et 2, **caractérisé en ce que** l'insert (7) est divisé par le joint coulissant (10) et le joint coulissant (12) avec le porte-aiguille (20) en trois chambres (9, 11, 15).

6. L'auto-injecteur selon la revendication 5, **caractérisé en ce que** la chambre supérieure (9) contient un anticonvulsivant, la chambre intermédiaire (11) contient un agent anticholinergique et la chambre inférieure (15) contient un réactivateur de cholinestérase.

7. L'auto-injecteur selon l'une quelconque des revendications précédentes, **caractérisé en ce que** la chambre inférieure (15) contient un réactivateur de cholinestérase sous forme dissoute.

8. L'auto-injecteur selon les revendications 1 à 6, **caractérisé en ce que** la chambre inférieure (15) contient un réactivateur de cholinestérase sous forme cristalline.
